# EUROPEAN PATENT SPECIFICATION

(11) **EP 2 914 593 B1**
(45) Date of publication and mention of the grant of the patent: **11.01.2017**
(21) Application number: 12786801.6
(22) Date of filing: 05.11.2012
(51) Int. Cl.: C07D 451/10, A61K 31/46, A61P 11/06

(54) **STABILIZATION OF TIOTROPIUM SOLVATES**
STABILISIERUNG VON TIOTROPIUMSOLVATEN
STABILISATION DE SOLVATES DE TIOTROPIUM

(43) Date of publication of application: 09.09.2015
(73) Proprietor: Zentiva, k.s., 102 37 Praha 10 (CZ)
(72) Inventor: CERNA, Igor, 169 00 Praha 6 (CZ); HAJICEK, Josef, 250 81 Nehvizdy (CZ); DAMMER, Ondrej, 253 01 Hostivice (CZ); KEBBATI, Mokhtar, F-93600 Aulnay Sous Bois (FR); BILLOT, Pascal, F-93100 Montreuil Sous Bois (FR); HOSEK, Patrik, F-77300 Fontainebleau (FR)
(74) Representative: Jirotkova, Ivana
(86) International application number: PCT/CZ2012/000112
(87) International publication number: WO 2014/067499

(56) References cited:
- WO-A2-2006/117299
- WO-A2-2006/117300
- WO-A2-2010/101538

## Description

### Technical field

The invention relates to a method of stabilizing solvates of tiotropium bromide and to stable solvates thus obtained.

### Background of the invention

Tiotropium bromide is an active ingredient with therapeutical benefit in the treatment of asthma or chronic obstructive pulmonary disease (COPD) and it was firstly described in the basic patent EP418716.

The chemical name of tiotropium bromide is (1α,2β,4β,7β)-7-[(hydroxidi-2-thienylacetyl)oxy]-9,9-dimethyl-3-oxa-9-azoniatricyclo[3.3.1.02,4]nonane bromide and it has following structure

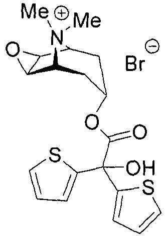

Tiotropium bromide is marketed by Boehringer-Ingelheim in an inhalation device under trade mark Spiriva. In the formulation it is present as the monohydrate form which was described in EP1326862. Tiotropium bromide is used in very small dosing; therefore it is necessary to micronize the active ingredient before or during processing to the formulation.

Tiotropium bromide is known in many polymorphic forms like anhydrous forms from WO2003000265, WO2006117299 or WO2005042527.

In literature there are described also solvates with various solvents. During the purification were prepared methanol/acetone solvates or dichloromethane/acetonitrile solvates in examples from the basic patent. Many solvates were mentioned in WO2006117299 and WO-2006117300. There were also prepared solvates with ethanol, methanol, 1,4-dioxane a 1,2-propanediol. The document WO2007075858 deals with, e.g., n-propanol solvate.

Dichloromethane solvate was mentioned in WO2011015883, n-butanediol solvate and allylalcohol solvates in WO2010101538.

According to previous documents the solvates are mainly used for isolation and as starting material for other forms for pharmaceutical formulation.

Documents WO-2006117299 and WO-2006117300 proposed using solvates in inhalable powders or aerosols, but in examples only an anhydrous form is used; there are no data about stability of formulation with solvates.

A formulation with solvates and lactose is described in WO2010101538, but there is no indication about stability of such formulation.

As was mentioned in WO2006117299 and WO2006117300 documents, the correct manufacture of the inhalable compositions which are suitable for use for the administration of a pharmaceutically active substance by inhalation is based on various parameters which are connected with the nature of the active substance itself. In the pharmaceutical compositions which are used, like tiotropium bromide, in the form of inhalable powders or in halable aerosols, the crystalline active substance is used in a micronized form for preparation of the formulation. The pharmaceutical quality of a pharmaceutical formulation requires that the active substance should have the same crystalline modification for the whole shelf life. The stability and properties of the crystalline active substance are subject to stringiest requirements from this point of view.

It is particularly desirable that the active substance be prepared in the form of a uniform and clearly defined crystalline modification. It is also particularly desirable that the active substance be prepared in a crystalline form which is characterized by a high degree of stability even over long storage periods.

There constantly exist a need for the stable forms of tiotropium bromide which are stable during micronization and during the shelf life of a prepared formulation.

### Summary of the invention

The present invention provides a method for stabilization of solvates of tiotropium bromide.

The solvates of tiotropium bromide, selected from solvates with methanol, ethanol, 2-propanol, 1,2-propanediol, 1,3-propanediol, n-butanol, 1,4-butandiol and *tert*-butanol, which are micronized or milled are deposited in a saturated atmosphere of the adequate solvent for the time period sufficient for recovery of the solvent. Thus, in the stabilization according to the invention, such time period is applied which is sufficient for recovery of the original amount of the solvent in the solvate.

Said solvates of tiotropium bromide are solvates with lower C1 -C4 alcohols, for example, with methanol, ethanol, 2-propanol, 1,2-propanediol, 1,3-propanediol, n-butanol, 1,4-butandiol, or *tert*-butanol.

Micronised or milled solvates have particle distribution D50 in the range of 1-10 µm measured by optical microscopy or Scanning Electronic Microscopy (SEM), preferably 3-5 µm. D50 means that 50% of the particles in a sample are of a size below the given value. Particle size was performed by an optical evaluation of several photos from optical microscopy (Nikon Eclipse E600 equipped with video camera and Archimed software) or SEM (Hitachi TM-1000, samples gold coated).

A tray with the adequate alcohol is placed near the micronized sample in a closed container to achieve saturated atmosphere. Vacuum can be applied in the range from 5 mbar to 100 mbar during a few minutes to fasten the process).

The adequate alcohol is the same alcohol which is incorporated in solvate. For example, for ethanol solvate ethanol atmosphere is used and for 1,3-propanediol solvate 1,3-propanediol atmosphere is used.

A time sufficient for recovery is commonly at least 1 hour, up to several days, preferably from 48 to 60 hours.

Stabilization is carried out preferably at a temperature in the range from 10 to 60°C, preferably at room temperature. Room temperature means a temperature in the range of 20-25°C.

Amounts of solvents are analysed by Gas Chromatography (GC). A RTX200 column or equivalent is used, injection temperature 130°C, 0.5µl injection with split 1/20°, detection by FID, detector temperature 280°C. The gas is Helium at a flow rate of 1.7 ml/hour. The temperature gradient is as given in Table 1:

**Table 1. Gas Chromatography temperature gradient**

| Initial Temperature [°C] | Final Temperature [°C] | Slope speed [°C/min] | Holding time [min] |
|---|---|---|---|
| 35 | 35 | Isothermal | 3.5 |
| 35 | 90 | 15 | 0 |
| 90 | 120 | 20 | 0 |
| 120 | 160 | 40 | 1 |
| 160 | 240 | 40 | 0.83 |

Solvates before stabilization are milled in stainless steel or Teflon jet mill, the target of 5µm was achieved by using, for example, the following parameter set, appr. feed rate 66 g/h, milling pressure 4 bar, injection pressure 5 bar.

In another embodiment the invention provides the new stable solvate of tiotropium bromide with 1,3-propanediol.

The invention relates to the crystalline tiotropium bromide solvate with 1,3-propanediol that has powder X-ray diffraction spectrum having main peaks (+/-0,2° 2Theta) 11,0; 15,3; 18,0; 21,4; 25,0, measured using CuKα, and other characteristic peaks 9,9; 13,4; 16,3; 19,8; 20,9; 23,5; 23,9; 24,6; 25,8; 26,0; 27,0; 27,8; 31,8.

In another embodiment the invention relates to a process for preparation of 1,3- propanediol solvate. The solvate can be prepared from any known form of tiotropium bromide via crystallization from 1,3-propanediol.

Tiotropium bromide is dissolved in pure 1,3-propanediol or a 1,3-propanediol mixture with miscible solvents at a temperature in the range from 40°C to the boiling point of the solvent or the solvent mixture, preferably in the range of 80-85°C. The solution can be concentrated, e.g., under vacuum. The solution is then cooled to a temperature in the range from 0 to 20°C. The 1,3-propanediol solvate is filtered off.

For further processing to a pharmaceutical formulation the active ingredient has to be micronized. In preformulation studies we tested the stability of the micronized API in long-term stability testing (25°C/60% relative humidity (RH)) and accelerated stability testing (40°C/75%RH). Ethanol solvate was stable during micronization as shown in Table 1. But both modes of stability testing showed low polymorphic stability; the solvate tends to convert to monohydrate form M or anhydrous form B1. The increased temperature caused increase in velocity of the polymorphic changes as shown in Table 2.

In preformulation studies there was also tested stability of the solvate in admixture with lactose which is used in inhalable powders, e.g., Lactohale LH 200. The testing confirmed the tendency to polymorphic changes - cf. Tables 5 and 6.

We have surprisingly discovered that stabilization of micronized solvates according to the invention solves the problem of polymorphic unstability of the solvates. The long-term and accelerated stability testing of the micronized solvates and their mixtures with lactose showed that the stabilized solvates possess an excellent polymorphic stability and are suitable for further processing to inhalable pharmaceutical formulations.

Control experiments were performed to evaluate the stability of solvate form of tiotropium bromide (ethanol solvate) during micronization (or milling). Micronization of this solvate form is feasible in a stainless steel jet mill. The target of 5 µm could be achieved by low energy milling. The particle size (evaluated by SEM) obtained was less than 3µm for the majority of particles and less than 6 µm for almost all particles by micronization (starting from from 50 µm to 700 µm for an unmilled sample).

It was observed that the micronization of the solvate form of tiotropium (ethanol solvate) led to small decrease in the content of residual solvent (Table 2). According to XRPD, only minor differences are present at 20° and 28°.

**Table 2. Comparison of ethanol solvate samples after micronization and stabilization**

| Sample | Ethanol content [GC] | TGA loss on weight | XRPD | Raman |
|---|---|---|---|---|
| Unmilled | 4.6 % w/w | 4.9 % w/w | Solvate form | Solvate form |
| Micronized | 4.2 % w/w | 4.3 % w/w | unchanged | unchanged |
| Stabilized | 4.5 % w/w | 5.1 % w/w | unchanged | unchanged |

It is shown in Table 2 that after micronization the content of ethanol in the micronized sample decreases (measured by GC). The decrease is about 0.4% of ethanol. After stabilization the content of ethanol measured with the same method is returned back to the original content 4.5%. The same behavior was observed also for 1,3-propanediol solvate.

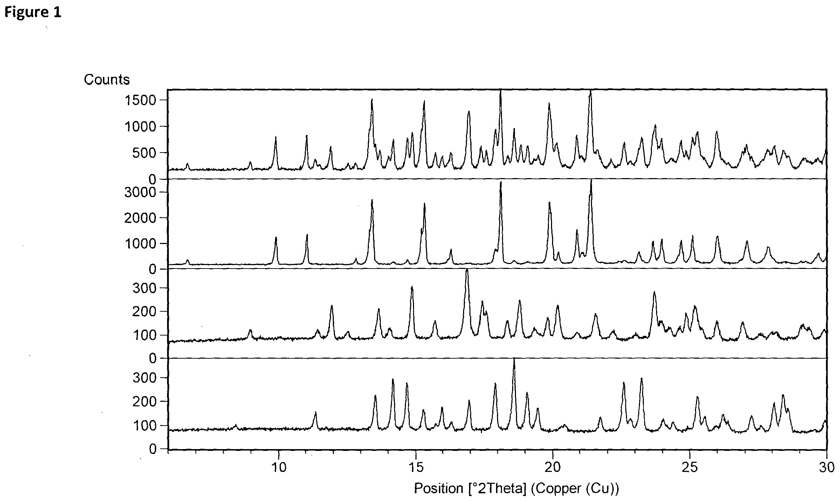

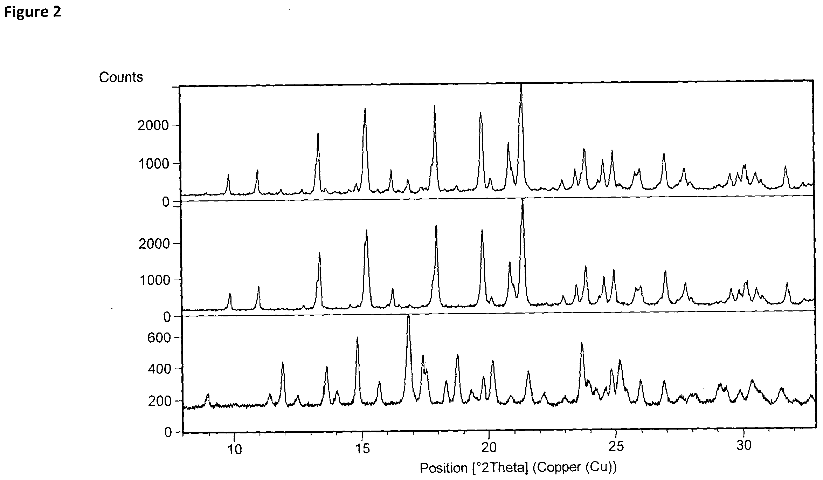

The polymorphic stability of non-stabilized and stabilized micronized solvates (both ethanol and 1,3-propanediol solvates) was evaluated under storage conditions (general case) in two modes including long-term (25°C/60%RH) and accelerated (40°C/75%RH) tests that test thermal stability and sensitivity to moisture. The polymorphic stability was controlled using XRPD. The results are summarized in Tables 2 and 3 and Figures 1 and 2 for each solvate.

**Table 3. Ethanol solvate**

| storage conditions | Time period (month) | non-stabilized | stabilized |
|---|---|---|---|
| long-term (25°C/60%RH) | 1 | unchanged | unchanged |
| long-term (25°C/60%RH) | 3 | unchanged | |
| accelerated (40°C/75%RH) | 1 | partial conversion to M and F-B1 | unchanged |

| | | | |
|---|---|---|---|
| M - monohydrate form; **F-B1 -** anhydrous form B1 | | | |

A partial phase transformation of non-stabilized micronized sample to a mixture of monohydrate form and anhydrous form is clearly visible. Stabilized micronized sample remains unchanged.

**Table 4. 1,3-propanediol solvate**

| storage conditions | Time period (month) | non-stabilized | stabilized |
|---|---|---|---|
| long-term (25°C/60%RH) | 1 | unchanged | unchanged |
| long-term (25°C/60%RH) | 3 | unchanged | unchanged |
| accelerated (40°C/75%RH) | 1 | unchanged | unchanged |
| accelerated (40°C/75%RH) | 3 | partial conversion to M | unchanged |

| | | | |
|---|---|---|---|
| M - monohydrate form; **F-B1 -** anhydrous form B1 | | | |

A partial phase transformation of non-stabilized micronized sample to monohydrate form is clearly visible. Stabilized micronized sample remains unchanged.

Both micronized solvates (namely ethanol and 1,3-propanediol solvates), stabilized and non-stabilized, were also subjected to tests of polymorphic stability in the presence of Lactohale LH 200 (as the most potential excipient for inhaler formulation) under long-term (25°C/60%RH) and accelerated (40°C/75%RH) storage conditions. It evaluates thermal stability and sensitivity to moisture in the presence of lactose monohydrate. The ratio between tiotropium bromide and Lactohale LH 200 was chosen as 1:1 (by weight). The polymorphic stability was controlled using XRPD. The results are summarized in Tables 4 and 5 for each solvate.

**Table 5. Ethanol solvate, mixture with Lactohale LH 200**

| storage conditions | Time period (month) | non-stabilized | stabilized |
|---|---|---|---|
| long-term (25°C/60%RH) | 1 | unchanged | unchanged |
| accelerated (40°C/75%RH) | 1 | conversion to M and F-B1 | unchanged |

| | | | |
|---|---|---|---|
| M - monohydrate form; **F-B1 -** anhydrous form B1 | | | |

A partial phase transformation of non-stabilized micronized sample to a mixture of monohydrate form and anhydrous form B2 is present at accelerated storage conditions. Stabilized micronized sample remains unchanged for both long-term and accelerated storage conditions after 1 month.

**Table 6. 1,3-propanediol solvate, mixture with Lactohale LH 200**

| storage conditions | Time period (month) | non-stabilized | stabilized |
|---|---|---|---|
| long-term (25°C/60%RH) | 1 | unchanged | unchanged |
| long-term (25°C/60%RH) | 3 | unchanged | unchanged |
| accelerated (40°C/75%RH) | 1 | unchanged | unchanged |
| accelerated (40°C/75%RH) | 3 | partial conversion to M | unchanged |

Non-stabilized and stabilized micronized samples of 1,3-propanediol solvate of tiotropium bromide remain unchanged for both long-term and accelerated storage conditions after 1 month.

It can be clearly seen that the deposition of tiotropium bromide solvate form in a saturated atmosphere of the adequate solvent after micronization or milling can enhance its stability even in the presence of lactose monohydrate.

### Brief description of the drawings

**Figure 1****.** Illustration of XRPD patterns of ethanol solvate and its comparison with the monohydrate form and anhydrous form B2. The figure depicts, from top to bottom, XRPD patterns of ethanol solvate of non-stabilized micronized sample after 1 month at accelerated storage conditions (40°C/75%RH), stabilized micronized sample after 1 month at accelerated storage conditions and XRPD patterns of the monohydrate form and anhydrous form B2 of tiotropium bromide.
**Figure 2****.** Illustration of XRPD patterns of 1,3-propanediol solvate and its comparison with the monohydrate form. The figure depicts, from top to bottom, XRPD patterns of 1,3-propanediol solvate of non-stabilized micronized sample after 1 month at accelerated storage conditions (40°C/75%RH), stabilized micronized sample after 1 month at accelerated storage conditions and XRPD patterns of monohydrate form of tiotropium bromide.

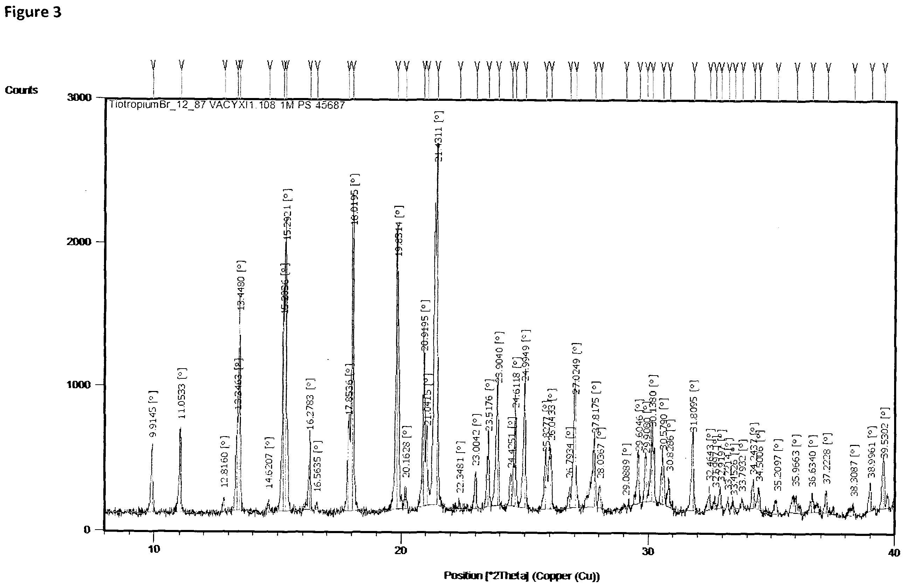

**Figure 3****.** Powder X-ray pattern of 1,3-propanediol solvate.

### Examples

### XRPD

*Device and conditions:* Brucker D8 advance, Reflecting mode, monocrystal carrier.

### Milling

*Device and conditions:* stainless steel jet mill, the target of 5 µm was achieved by using for example the following parameter set, appr. feed rate 66 g/h, milling pressure 4 bar, injection pressure 5 bar.

### Particle size measurement by Optical microscopy or by SEM:

*Device and conditions:* Scanning electron microscope (SEM) Hitachi TM-1000, Gold coated: particle size was performed by an optical evaluation of several photos from microscopy (Nikon Eclipse E600 equipped with video camera and Archimed software) and SEM.

### TGA

*Device and conditions:* TA instr. Q500, start at 25°C and heated with 10 K/min up to 280°C.

### Raman

*Device and conditions:* Kaiser Optical System RXN1, Phat-Sonde

### Example 1

Crude tiotropium bromide 4.0 g was dissolved in anhydrous ethanol (200 ml) at reflux. The stirred solution was cooled down to 15 °C during 2 hours. The obtained suspension was filtered and the solid washed with anhydrous ethanol. The product was dried at 30 °C under reduced pressure (100 mbar) for 24 hours. 3.5 g of tiotropium bromide ethanol solvate form were obtained (yield 87 %). (XRPD) solvate form; (DSC) Tonset1=154,1°C, Tpeak1=159°C major endotherm Tonset2=228,8°C, Tpeak2=230,8°C; (TGA) 5,4%.

### Example 2

Tiotropium bromide (dichloromethane/acetonitrile solvate) 4.0 g was dissolved in 96% ethanol (80 ml) at 95 °C. The stirred solution was cooled down to -10 °C during 45 min. The obtained suspension was stirred for another two hours. Suspension was filtered and the solid washed with minimum amount of anhydrous ethanol. The product was dried at 50 °C under reduced pressure (25 mbar) for 24 hours. 3.4 g of tiotropium bromide ethanol solvate form were obtained (Yield 86 %). (XRPD) solvate form.

### Example 3

In a 1 liter heated vessel, 100 g of tiotropium bromide are dissolved at 80 to 85°C in 600 ml of 1,3-propanediol. Around 100 ml are distilled off under slight vacuum; the distillation can be stripped with gas. The mixture is then cooled down to 5°C to allow crystallization and filtered. The cake is washed with 100 ml of 1.3-propanediol 2 times and dried under vacuum (around 40 mbar) at 40°C.

### Example 4 - Stabilization of an ethanol solvate

After jet-milling, the recovered product is placed in a tray as a thin layer. On the bottom of a desiccator, under the perforated plate, a tray containing liquid ethanol, typically 100 ml of ethanol, is placed. The sample, 0.2 g as thin layer is placed in the desiccator, on the perforated plate. The desiccator is then tightly closed and vacuum is applied (40 mbar) during 10 minutes. The product is allowed to stabilize during 60 hours at room temperature. The product is then placed in a tight bottle.

### Example 5 - Stabilization of a 1,3-propanediol solvate

After jet-milling, the recovered product is placed in a tray as a thin layer. On the bottom of a desiccator, under the perforated plate, a tray containing liquid 1,3-propanediol, typically 100 ml of 1,3-propanediol, is placed. The sample, 1 g as thin layer is placed in the desiccator, on the perforated plate. The desiccator is then tightly closed and vacuum is applied (40 mbar) during 10 minutes. The product is allowed to stabilize during 60 hours at room temperature. The product is then placed in a tight bottle.

### Example 6 - Stabilization of a 1,3-propanediol solvate

After jet-milling, the recovered product is placed in a tray as a thin layer. On the bottom of a desiccator, under the perforated plate, a tray containing liquid 1,3-propanediol, typically 100 ml of 1,3-propanediol, is placed. The sample, 1 g as thin layer is placed in the desiccator, on the perforated plate. The desiccator is then tightly closed and Vacuum is applied (40 mbar) during 10 minutes. The product is allowed to stabilize during 60 hours at 40°C. The product is then placed in a tight bottle.

### Example 7

Methods of testing stability of solvates with or without lactose.

### Preparation of samples of tiotropium bromide solvate for natural and accelerated stability testing

50 mg of a Tiotropium bromide solvate form (ethanol or 1,3-propanediol solvate) was transferred to a PE foil zipper-sealed bag. This bag was inserted in another PE foil zipper-sealed bag and finally, the bag was packed in an Al Foil bag and sealed. These bags were stored under specified conditions for natural (25°C/60%RH) or accelerated (40°C/75%RH) stabilities.

### Preparation of samples of tiotropium bromide solvate for natural and accelerated stability testing with lactose

100 mg of Tiotropium Bromide and 100 mg of Lactohale LH 200 (milled alpha-lactose monohydrate pharmaceutical) were transferred to a PE foil zipper-sealed bag, and mixed together. This bag was inserted in another PE foil zipper-sealed bag and finally, the bag was packed in an Al Foil bag and sealed. These bags were stored under specified conditions for natural (25°C/60%RH) or accelerated (40°C/75%RH) stabilities.

## Claims

1. A method for stabilization of micronized or milled solvates of tiotropium bromide, selected from solvates with methanol, ethanol, 2-propanol, 1,2-propanediol, 1,3-propanediol, n-butanol, 1,4-butandiol and *tert*-butanol, **characterized in that** the solvates are deposited in a saturated atmosphere of the adequate solvent for the time period sufficient for recovery of solvent.

2. The method according to claim 1, **characterized in that** said solvates have a D50 in the range of 1-10 µm.

3. The method according to claim 1, **characterized in that** said solvates are deposited in close container where vacuum is applied to achieve saturated atmosphere of the adequate solvent.

4. Tiotropium bromide solvate with 1,3-propanediol.

5. Tiotropium bromide solvate according to claim 4, which has a powder X-ray diffraction spectrum having the main peaks (+/-0,2° 2Theta) 11,0; 15,3; 18,0; 21,4; 25,0, measured using CuKα.

6. Tiotropium bromide solvate according to claim 5, which has a powder X-ray diffraction spectrum having the other characteristic peaks (+/-0,2° 2Theta) 9,9; 13,4; 16,3; 19,8; 20,9; 23,5; 23,9; 24,6; 25,8; 26,0; 27,0; 27,8; 31,8.

7. A method of preparing the tiotropium bromide solvate according to claims 4-6, **characterized in that** tiotropium bromide is dissolved in 1,3-propanediol at a temperature in the range from 40°C to the boiling point, the solution is cooled to a temperature in the range from 0°C to 10°C and the solid solvate is filtered off.

## Patentansprüche

1. Verfahren zur Stabilisierung von mikronisierten oder gemahlenen Solvaten von Tiotropiumbromid, ausgewählt aus Solvaten mit Methanol, Ethanol, 2-Propanol, 1,2-Propandiol, 1,3-Propandiol, n-Butanol, 1,4-Butandiol und *tert*-Butanol, **dadurch gekennzeichnet, dass** die Solvate in eine gesättigte Atmosphäre des adäquaten Lösungsmittels für die Zeitdauer eingebracht werden, welche für die Rückgewinnung des Lösungsmittels ausreicht.

2. Verfahren nach Anspruch 1, **dadurch gekennzeichnet, dass** die Solvate einen D50-Wert im Bereich von 1-10 µm aufweisen.

3. Verfahren nach Anspruch 1, **dadurch gekennzeichnet, dass** die Solvate in einen geschlossenen Behälter einge-bracht werden, in welchem ein Vakuum angelegt wird, um eine gesättigte Atmosphäre des adäquaten Lösungsmittels zu erzielen.

4. Tiotropiumbromidsolvat mit 1,3-Propandiol.

5. Tiotropiumbromidsolvat nach Anspruch 4, das ein Pulver-Röntgenbeugungsspektrum mit den Hauptpeaks (+/-0,2° 2Theta) 11,0, 15,3, 18,0, 21,4, 25,0, gemessen unter Verwendung von CuKα, aufweist.

6. Tiotropiumbromidsolvat nach Anspruch 5, das ein Pulver-Röntgenbeugungsspektrum mit den anderen charakteristischen Peaks (+/-0,2° 2Theta) 9,9, 13,4, 16,3, 19,8, 20,9, 23,5, 23,9, 24,6, 25,8, 26,0, 27,0, 27,8, 31,8 aufweist.

7. Verfahren zur Herstellung des Tiotropiumbromidsolvats nach den Ansprüchen 4-6, **dadurch gekennzeichnet, dass** Tiotropiumbromid in 1,3-Propandiol bei einer Temperatur im Bereich von 40 °C bis zum Siedepunkt gelöst wird, die Lösung auf eine Temperatur im Bereich von 0 °C bis 10 °C abgekühlt wird und das feste Solvat abfiltriert wird.

## Revendications

1. Un procédé de stabilisation de solvates micronisés ou broyés de bromure de tiotropium, choisis parmi les solvates formés avec le méthanol, l'éthanol, le 2-propanol, le 1,2-propanediol, le 1,3-propanediol, le n-butanol, le 1,4-butanediol et le tert-butanol, **caractérisé en ce que** les solvates sont déposés dans une atmosphère saturée du solvant approprié pendant la période de temps suffisant pour récupérer le solvant.

2. Le procédé selon la revendication 1, **caractérisé en ce que** lesdits solvates ont un D50 de l'ordre de 1 à 10 µm.

3. Le procédé selon la revendication 1, **caractérisé en ce que** lesdits solvates sont déposés dans un conteneur fermé, où l'on applique le vide, pour obtenir une atmosphère saturée du solvant approprié.

4. Le solvate de bromure de tiotropium formé avec le 1,3-propanediol.

5. Le solvate de bromure de tiotropium selon la revendication 4, qui présente un spectre de diffraction des rayons X sur poudre présentant les pics principaux (2 thêta ± 0,2°) pour 11,0; 15,3; 18,0; 21,4; 25,0, mesurés à l'aide de CuKα.

6. Le solvate de bromure de tiotropium selon la revendication 5, qui présente un spectre de diffraction des rayons X sur poudre présentant d'autres pics caractéristiques (2 thêta ±0,2°) pour 9,9; 13,4; 16,3; 19,8; 20,9; 23,5; 23,9; 24,6; 25,8; 26,0; 27,0; 27,8; 31,8.

7. Un procédé de préparation de solvate de bromure de tiotropium selon les revendications 4 à 6, **caractérisé en ce que**
- le bromure de tiotropium est dissous dans du 1,3-propanediol à une température comprise dans l'intervalle de 40°C et le point d'ébullition,
- la solution est refroidie à une température comprise dans l'intervalle de 0°C à 10°C et
- le produit de solvatation solide est séparé par filtration.
